# EUROPEAN PATENT SPECIFICATION

(11) **EP 0 548 157 B1**
(45) Date of publication and mention of the grant of the patent: **20.05.1998**
(21) Application number: 91916129.9
(22) Date of filing: 13.09.1991
(51) Int. Cl.: A61K 47/48, A61K 49/00

(54) **USE OF PARTICULATE AGENTS**
VERWENDUNG VON SUBSTANZEN IN PARTIKELFORM
UTILISATION D'AGENTS SOUS FORME DE PARTICULES

(30) Priority: 14.09.1990 GB 9020075; 30.10.1990 GB 9023580; 17.12.1990 GB 9027293; 07.01.1991 GB 9100233; 16.01.1991 GB 9100981; 31.01.1991 GB 9102146; 20.05.1991 GB 9110876; 30.07.1991 GB 9116373; 19.08.1991 GB 9117851; 30.08.1991 GB 9118676
(43) Date of publication of application: 30.06.1993
(60) Divisional application: 97119199.4
(73) Proprietor: SYNGENIX LIMITED, Cambridge CB3 0BL (GB)
(72) Inventor: FILLER, Aaron, Gershon, London SW20 0NE (GB)
(74) Representative: Perry, Robert Edward
(86) International application number: EP9101780
(87) International publication number: WO9204916

(56) References cited:
- WO-A-86/01112
- WO-A-88/00060
- WO-A-89/09625
- WO-A-90/01295
- STN File Server, File Medline, accession no. 87239688; J.E. GALLAGHER et al.: "Sialic acid mediates the initial binding of positively charged inorganic particles to alveolar macrophage membranes"
- STN File Server, File Medline, accession no. 86085167; D. MENETREY: "Retrograde tracing of neural pathways with a protein-gold complex. I. Light microscopic detection after silver intensification"

## Description

This invention relates to particulate agents for use in therapy.

US-A-4849210 and US-A-4863715 demonstrate the effectiveness of suspensions of ferromagnetic particles as intravenous MRI contrast agents. WO-A-8800060 and US-A-4827945 provide a number of additional methods of synthesis of superparamagnetic particles and suggest the MR intravascular use of a wide range of labelled particles analogous to those disclosed for *in vitro* use in US-A-4452773.

The particles produced by these additional methods vary in size from 10 to 5000 nm (100 to 5,000 Angstroms). They are not useful for radionuclide imaging of axonal transport because most of the particles are too large to be endocytosed by neurons and only a small proportion will carry active targeting moiety.

Brady, SMRM 10:2 (1991), verbally reported transport of MR detectable particles after direct injection into the sciatic nerve; however he exhibited only an image of transport after the completely severed sciatic nerve was soaked in a gel with ferrite particles. Ghosh, SMRM 10:1042 (August 1991), similarly reported evidence of transport of ferrite particles after direct pressure injection into the brain of a frog, although no MR detection was achieved. Neither reference teaches how pharmaceutical use of axonal transport could be achieved since these techniques involved irreparable destruction of vital neural tissue. Intraneural injection is destructive of the nerve at the site of needle puncture and causes forced flow of tracer in the nerve sheath which may actually mask evidence of actual axonal transport. Madison, Brain Res. 522:90-98 (1990), also reported pressure injection of latex nanospheres into the brain, to deliver toxic agents for the killing of neurons after subsequent photoactivation.

Non-destructive administration of toxic anthracycline antibiotics has been reported, but this was done to study the chemical nature of the neural uptake process and the fluorescent effect of the agents rather than to achieve any therapeutic effect, and the agents concerned were neurotoxic; see England, Brain 111:915-926 (1988), and Bigotte, Neurology 37:985-992 (1987).

The existence of axonal transport (or 'axoplasmic flow') has been known for over 40 years and it has been known for twenty years that certain foreign materials injected into muscle would be endocytosed (swallowed up) by the axon terminus and then subsequently be detectable in the neuron cell body; however, until the developments described herein, all methods of detection have required lethal interventions, generally requiring the killing of the experimental animal with subsequent specialized tissue processing.

A series of relatively non-specific substances for uptake were tried including Evans-Blue stain conjugated to albumin and also horseradish peroxidase (HRP) enzyme, and radio-labelled amino acids for anterograde labelling. The principle of improving specificity and uptake efficiency of a histologically identifiable tracer was taken further by Schwab (Brain Res. 130:190-196 (1977)) who attached nerve growth factor (NGF) to HRP. It was also Schwab who showed that a plant lectin called wheat germ agglutinin (WGA) was an excellent nerve adhesion molecule and again Schwab who introduced the use of viral fragments and toxins as labels (see Brain Res. 152:145-150 (1978) and J. Cell Biol. 82:798-810 (1979)).

WGA conjugated to HRP was later suggested as a tracer and this one agent has been the predominant agent of choice in many hundreds of subsequent studies involving axonal transport. The conjugation to some post-sacrifice visualization moiety such as HRP permitted the use of a chromogen histochemical staining reaction. Other means of visualization of tracers included autoradiographic histology or immunocytochemical techniques.

The present invention is based on the realisation that the endocytosis of nerve adhesion molecule (NAM)-labelled agents can be utilised for the remote delivery of therapeutic agents, i.e. axoplasmic flow can serve to transport a therapeutically-active agent comprising a nerve adhesion moiety from its administration site in tissue such as muscle to a remote site where it exerts its pharmacological effect. This is of particular interest where the sensitivity or accessibility of the remote site is such as to prevent direct administration of the pharmaceutical.

According to the present invention, a therapeutic agent comprises particles having, coupled thereto, a nerve adhesion moiety and a physiologically-active substance, the nerve adhesion moiety serving to promote neuronal endocytosis of said agent, the agent being capable of axonal transport following neuronal endocytosis, and is used for the preparation of a medicament for use in providing the therapeutic effect of the physiologically-acceptable substance on administration into a vascularised peripherally innervated tissue site or into other tissue sites innervated by a spinal root.

The pharmaceutical agent is preferably administered into a tissue site, such as a muscle, having a volume of at least ten times that of the group of nerve cells which are to transport the agent.

The particulate NAM-coupled moiety may be coated or uncoated. If coated, the coating may be selected to be broken down within the neuron after endocytosis, either slowly or more rapidly, or to be maintained during axonal transport.

For the purposes of the present invention it should be appreciated that while natural or synthetic, essentially inert, organic polymer particles (such as dextran-coated microspheres or latex nanospheres) are capable of being endocytosed, these organic polymers unlike more specific and complicated molecules such as proteins, antibodies and antibody fragments are not considered to be nerve adhesion molecules.

The mean particle size for the particulate pharmaceutical agents used in the invention is conveniently in the range 5 to 100 nm, especially 8-70 nm, more particularly 10 to 50 nm and preferably about 20-30 nm.

One especially important group of pharmaceutical agents for use according to the invention is that of NAM-coupled particulate inorganic compounds, for example metal oxides, sulphides or alloys, where the inorganic material is selected for its magnetic properties, in particular ferri- and ferromagnetism and more particularly superparamagnetism, or includes within an otherwise essentially inert matrix atoms or molecules which are released gradually from the matrix to exert a therapeutic or prophylactic effect. Many metal oxide structures may be utilized as the inorganic particles, and spinels and garnets have been found to be particularly useful in this regard. It should however be stressed that other well known inert and preferably essentially water-insoluble metal compounds may be used, especially those having or capable of being doped to exhibit cooperative magnetic properties and those having lattices such as permit desired radioisotopes to be included. By alloys, mixed metals are of course included. Organic particulate matrices may also be used to accommodate a therapeutic compound.

As is clear from the above, this invention is especially concerned with improvements in particulate pharmaceuticals which surprisingly result in providing access to new patterns of distribution within the body which were never previously possible. As a result of these new patterns of distribution, a number of previously intractable problems in medical diagnosis and treatment have now been solved.

For the particulate pharmaceutical agents in particular, the improvements include 1) improved control of particle size, 2) development of an effective means of affinity purification of the particles, 3) demonstration of a means of filter sterilization of the concentrated product late in the synthesis, and 4) widening the array of elements and concentration of those elements applied to medical uses in metal oxide or sulphide crystals or in alloys.

One of the most important consequences of these advances is the development of agents which can be delivered by, and make use of an entirely novel intraneural pharmaceutical route (IPR). In addition, these advances greatly simplify and reduce the cost of production of related particulate agents with previously known uses.

In a preferred embodiment, the agent comprises particles with a core metal oxide crystal, eg. of spinel or garnet structure, coated for example by dextran carbohydrate wherein the total size of the coated particle is between 100 and 500 Angstroms (10 to 50 nanometers) and where a targeting moiety (TM) is chemically bound to the coating in low concentration of TM per particle, preferably 1:1. The agent should preferably be virtually free of particles lacking an active TM, and compositions thereof are preferably sterilized by 0.2 or 0.1 micron microfiltration after final synthesis, affinity purification and concentration.

The uses of a given version of the agent depend upon the elements and isotopes (nuclides) used in the initial precipitation step in which the metal oxide crystal core is precipitated and coated and also upon the type of targeting moiety used. For each use, the nuclide and targeting moiety may be selected to benefit both from the general advantages of the simplicities of the preparatory method and to take advantage of the new types of pharmaceutical distribution which can be achieved by materials prepared in this way.

The inorganic particles in the preferred pharmaceutical agents used according to the invention generally fall into one of two categories:
i) particles incorporating a gamma or electron-emitting radionuclide, and thus being capable of causing radiation treatment effects; or
ii) particles incorporating a compound or element capable on release, e.g. during degradation of the particle, of effecting a desired therapeutic or prophylactic effect.

Metal oxide particles of the first type are clearly known. Particles of the latter type are novel.

The invention may utilise a physiologically-tolerable particulate garnet or spinel with incorporated therein atoms of scandium, of a radioactive yttrium isotope, of a sixth period metal (e.g. a lanthanide), of a high MR receptivity nuclide (e.g. at least as high as ₇₁Lu¹⁷⁵), or of an element which on particle degradation has a desired therapeutic or prophylactic activity.

It will be appreciated that although the metals of the metal oxide, sulphide or alloy matrices of the particles of the invention may have naturally-occurring positron emitting isotopes the particles according to the invention have significantly higher than natural abundance contents of these, e.g. for positron emitters an average of at least one, perhaps 10 or more atoms per 100 nm crystal. The natural occurrence of many β⁺ emitters is less than 1 in 10²⁰ and even one emitting atom per particle may suffice.

For other novel "doped" particles according to the invention, the active or marker nuclei may be isotopes which occur naturally, e.g. as impurities in naturally occurring oxides, sulphides or alloys - in this case again the particles according to the invention are distinguished by containing such atoms at higher than natural values, e.g. a hundred or even more per 100 nm particle.

The particles of the invention may be coated or uncoated and may derive their physiological tolerability at least in part from such a coating. They are moreover coupled to a biotargetting moiety, for example an antibody, an antibody fragment or another NAM.

The particles of type i) mentioned above are also preferably of a spinel or garnet structure - the manufacture of particles of these types is already well known and need not be described further here. By way of interest however it may be noted that superparamagnetic crystals of this type have been proposed for use as MRI contrast agents in various patent publications of Nycomed AS, Schering AG, Advanced Magnetics Inc, etc (eg. US-A-4863715 (Jacobsen) and US-A-4827945 (Groman)).

There are a wide variety of targeting moieties or NAMs which can be used according to the invention. These include antibodies, monoclonal antibodies, antibody fragments, receptors, peptides such as endorphins, steroid molecules, viral fragments or coat proteins, cell surface antigens including various carbohydrates, lectins, immunoadhesins, neurotransmitter molecules, growth factors, and other proteins which promote endocytosis of the pharmaceutical agent by the axon termini. The use of lectins, such as WGA, is particularly preferred.

The particular agents used in the invention preferably comprise therapeutically or prophylactically-loaded inorganic crystals, e.g. β⁺ emitter-marked metal oxides.

There are a number of nuclides emitting positron and electron β-particles, all of which can be included in metal oxides, e.g. spinels such as ferrites, either as substituents in the crystal lattice or as seeds, e.g. ZrO₂, inside ferrite spinel shells. This provides a new and unique way of delivering these various nuclides to various medically useful locations in the body in a wide variety of new concentrations and half lives. A single ferrite particle can be used to attach hundreds or thousands of β-emitting atoms to a single antibody, thus far exceeding the intensity of signal per antibody molecule available in current preparations which generally provide one emitting atom per antibody molecule.

The uses for these various beta-emitting ferrites include a number of treatment modalities. From one point of view, this array of possible nuclide preparations presents a wide range of half lives and particle energies which can be used for various tasks. The great simplification provided is that all of these can be manufactured and delivered by means of essentially similar molecules with effectively identical chemistry. Most of these nuclides decay to daughters which are also easily accommodated in the crystal and therefore do not involve loss of integrity of the particle as decay progresses.

In one set of embodiments, the positron-emitting isotopes of manganese (₂₅Mn⁵²), iron (₂₆Fe⁵²), cobalt (₂₇Co⁵⁵), or rhodium (₄₅Rh⁹⁹) are used in the synthesis of spinel particles, eg. sub-domain sized, superparamagnetic ferrite particles. The inclusion of cobalt or manganese in this type of ferrite has previously been difficult to achieve efficiently, but it is possible to reliably introduce cobalt, manganese, or other metals in amounts up to 1/3 of the number of metal atoms per formula unit, e.g. with the remaining 2/3 being Fe(III) if the stoichiometry of the desired crystal structure, e.g. garnet or spinel, is carefully considered and factors such as pH, temperature, and precursor metal salt and coating compound concentrations and the duration of heat incubation after precipitation are carefully controlled, preferably after optimization by routine experimentation. Thus as an example, for dextran coated particles it has generally been found advantageous to precipitate out from a saturated dextran solution. Thus all the divalent metal atoms may be replaced as opposed to the ½ or fewer suggested by Groman in US-A-4827945.

These particles may be synthesized in such a way that they are stably coated with dextran or other hydrophilic molecules and the coating may then be activated and bound covalently to antibodies or any type of nerve adhesion molecule.

Transition metal or lanthanide nuclides can be used in the synthesis of radioactive metal compounds (e.g. a metal oxide, metal sulphide or alloy, such as a ferrite) for use in monoclonal antibody-based treatment of tumours by irradiation. Here again, the biodistribution and clearance of the delivered radionuclides are quite different from single atoms chelated to the proteins. Intravascular injection of Fe⁵⁹-labelled particles has demonstrated a biphasic plasma half-life with about ¾ of the dose being cleared to spleen, liver, marrow, and slightly to lung over 1-2 hours, but with a substantial fraction of the dose demonstrating a quite prolonged plasma half life of many hours. Each antibody molecule can be used to deliver several hundred or several thousand atoms of the desired nuclide so achieving a high local dose. It should also be noted that binding multiple emitter atoms to a single protein molecule has been known to rapidly destroy the protein - this problem is substantially alleviated by the SMPE particles because the emitting nuclei are up to 100 angstroms distant from the NAM - thus the chance of any electron, positron, or gamma-ray interacting with the targeting NAM is reduced by several orders of magnitude. Methods developed for antibody delivery of ₃₉Y⁹⁰ can be applied with a far higher concentration of this nuclide included in a conjugated ferrite. Another treatment problem where these β-emitting ferrites could be useful is in improving the current methods of intra-articular radiotherapy in rheumatoid conditions.

Defects in a crystal can cause trapping of positrons. Defects in YBaCuOₓ perovskite crystals are particularly effective at positron trapping even when these materials are not in a superconducting state, however, even mechanical stress defects in metals are fairly effective. There are also effects due to the magnetic field generated by a moving positron and its interaction with the spontaneous field of a material such as magnetite, as well as electron interaction enhancement effects due to the number of unpaired, anti-spin matched electrons from d or f orbitals in the particular spinel used for the particulate shield.

The consequence of these considerations is that it is possible to begin with a crystal seed of a positron-emitting nuclide including several thousands atoms of the emitter and then to precipitate a lithium or zinc doped, defected, magnetite shield around the positron emitting core. This shield will cause a very large fraction of the emitted positrons to undergo all of their ionization producing collisional losses within the particle and therefore to annihilate without ever leaving the particle. Those positrons that do emerge from the surface of the particle without being affected by reflection or surface trapping effects will have a greatly reduced energy distribution, travel far shorter distances through tissue, and create far fewer ionizations in tissue per decay event than standard unshielded positron emitters.

This shielding can be used to achieve extremely limited ranges of cytotoxic ionization injury.

The range of the emitted β-particles can be designed to be not much greater than the size of a single target cell, thus limiting effective irradiation to only those cells that actually ingest the particle and taking advantage of the terminal Bragg peak effect which increases the ionization rate for a low energy positron just before annihilation. A short half life emitter could be used to minimize the effect of increasing exposure range with digestion of the coating (which may take days) and multiple treatments could then be carried out. Larger particles can be used without magnetic aggregation by composing the shell of less magnetic nuclides.

Metals other than β-emitters can be used to achieve the very short range radiotherapy effect. There are a variety of nuclides in which decay is by K-shell capture. An optimal nuclide with this behaviour is ₄₆Pd¹⁰³ which is a pure K-capture nuclide with a 17 day half-life; ₂₄Cr⁵¹ may also advantageously be used.

The particles used generally should be metal compounds capable of precipitation to a stable colloid having a particle size suitable for cell uptake and having a surface capable of being coated with or bound to biochemically useful materials, e.g. carbohydrates or proteins.

Particles for use in the invention may experience mechanical vibration and hence heating when subjected to resonant tuned microwave energy. Such particles may be transported into areas of spinal cord injury where the development of scar prevents the regeneration of injured spinal cord tissue. In research work, this localised heating phenomenon might be used as a means of inhibiting spinal cord scar formation. This effect may also be applied for selective tumour diathermy. Intramuscular injection at various sites with different resonant particle frequency types at each site will permit rotation of microwave heating frequencies so that only the tumour site will be stimulated by all the signals.

From the above, it will be appreciated that the invention provides an entirely novel means of pharmaceutical distribution which involves the entrainment of a well known physiological phenomenon called axonal transport. A central feature is that the total body distribution after intramuscular injection of the pharmaceutical agent quite unexpectedly yields dramatically high intraneural concentration relative to other tissues. This differential in body/nerve concentration permits the use of this route with relatively small amounts of pharmaceutical agent to achieve treatment effects.

Non-destructive administration of toxic anthracycline antibiotics has been reported, but this was done to study the chemical nature of the neural uptake process and the fluorescent effect of the agents rather than to achieve any therapeutic effect, and the agents concerned were neurotoxic (see England in Brain 111:915-926 (1988) and Bigotte in Neurology 37:985-992 (1987)).

Unlike any of these previous reports, the agents described herein may be introduced by techniques which do not involve the destruction of neural tissue and which then achieve a pharmacologic effect which does not require any toxic injury to neural tissues. By delivering particulate carriers it becomes possible to deliver types of pharmaceutical agents which would be irreparably damaged by direct chemical conjugation to a NAM or on break up of its direct NAM-conjugate within the cell. Instead the NAM is coupled to the particle and the drug is included in the particle or in the particle coating. Further, the use of particulate drug carriers permits the introduction of large numbers of molecules of the pharmaceutical agent with each endocytotic event thus yielding a 100 fold or up to one million fold increase of uptake efficiency per NAM. This amplification effect may be crucial to achieving pharmacologically efficacious doses in many situations. The methods of administration for these beneficial diagnostic and therapeutic uses include topical, intravenous, intrathecal/intracisternal (cerebro-spinal fluid), sub-cutaneous, intradermal, intra-nasal, eye-drop, or bladder irrigation methods, but intramuscular administration is to be preferred.

The agents described herein differ from all previously used axonal tracers in that they include agents capable of controlled administration by safe intramuscular injection with non-toxic substances and of achieving whole body distributions which permit their useful observation by various types of non-invasive imaging modalities. The agents may be biodegradable, safe for clinical use, and act to reveal various human disease conditions which cannot be adequately demonstrated by existing techniques.

Previous uses of axonal tracers have been concerned with optimizing the degree of post-sacrificial staining of the neuron cell body in brain or spinal cord. It has not previously been evident that useful concentrations and distributions of clinically applicable tracer materials could be achieved.

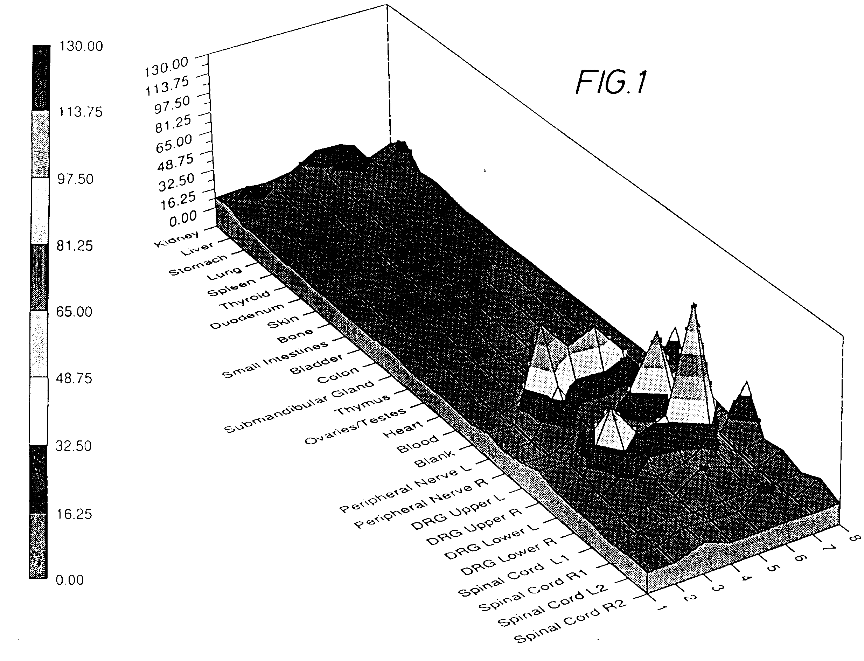

However, this set of agents is based on the discovery that when a nerve adhesion molecule which also has affinity for markers on the muscle cell surface is used, the injected material has very minimal spread from the site of intramuscular injection. In consequence, a relatively large amount of the substance is transported into the nerve while relatively little spreads throughout the body. The initial distribution assay results with animal studies using ¹²⁵I labelled WGA are shown in Fig. 1, the accompanying drawing. This graph depicts the results of the ¹²⁵I-WGA distribution study, in which the vertical axis gives counts per minute per gm of tissue normalised by dividing by the cpm/gm of blood for the individual animal in the series, various tissues are displayed along the long Y axis, and lines 1 through 8 reflect the results from the different animals with varying treatment/survival times and doses. DRG signifies spinal roots and dorsal root ganglia and demonstrates concentrations 5-10 times higher than any other tissue except the muscle and lymph nodes near the injection site which are not shown.

The delivery of particulate pharmaceuticals by the intraneural route is an entirely novel means of drug administration. The largest number of drugs in current use depend in some way upon the bloodstream to achieve their distribution. This vascular dependence includes not only drugs given by intravenous or intra-arterial routes, but also most orally administered drugs which must be absorbed into the bloodstream to reach target tissues, most intramuscularly administered drugs which are absorbed by the muscles blood vessels, many inhaled agents, most intranasally applied drugs, some rectally administered drugs such as paraldehyde, and many topically administered agents such as transdermal nitroglycerine. There are, however some drugs which are delivered into and distributed by the cerebrospinal fluid (intrathecal route), some oral drugs which are not absorbed (kaolin, oral vancomycin), a variety of topical and intravaginal agents, and some administered percutaneously for local effect or intraarticular effect such as local anaesthetics, and locally administered steroids.

Various new drug forms and methods of use described below involve delivery via an intraneural route. Access to this route may be obtained by oral ingestion, topical, intra-articular, intrathecal, intravenous and, preferably, intramuscular administration. However, common to all these new methods, is that the dosing and active site of the agent is determined by a route which involves endocytosis by nerve endings with subsequent transport to a different and distant part of the neuron.

In some experimental studies, various agents for transport have been introduced by intraneural injection or by application to the cut end of a severed nerve. In these methods, the 'blood brain barrier' due to the perineurium is traversed by mechanical injury, and much of the uptake of tracer is due to direct presentation at cut nerve endings where specific nerve adhesion molecules may be irrelevant. The intramuscular technique (and also the intravenous application) depend upon the natural defect in the perineurium which occurs at axon terminus. Thus the blood brain barrier is naturally incomplete at this site so that tracers reversibly adherent to muscle or emerging from small blood vessels passing near neuromuscular synapses can present a variety of molecules and particles directly to the neuronal cell surface for uptake after specific adhesion to the neuronal cell surface at the axon or dendrite terminus.

The site of injection or administration will preferably be determined only by knowledge of the nerves which project to that site. For example, a pain in the large toe will be known to the neurologist to involve the dorsal root ganglion of the fifth lumbar nerve root. He will then choose an injection site somewhere in the dermatome or myotome served by that nerve root in order to label the part of the nerve he believes to be impaired or to deliver, for instance, a pain medication to the ganglion or spinal cord dorsal root entry zone connected to the fifth lumbar spinal nerve root. For drugs where systemic spread is to be minimised, it is particularly important to be able to achieve high uptake by neurons per unit amount injected and to minimise spread away from the injection site.

The nerve adhesion molecules used to initiate the uptake and transport of the pharmaceutical agents used according to the invention all have in common with each other some tendency to promote uptake by neurons. Some molecules with no particular affinity may be taken up and transported inefficiently by neurons. However, molecules which interact with and bind to specific cell surface markers or receptors on the nerve ending of the selected nerve type are far more efficient and are preferred. An additional degree of efficiency can be obtained when the compound also has some affinity for the cell surface of muscle cells, since this will promote the depot effect at the injection site and decrease the tendency for the agent to diffuse away or be carried away by the bloodstream prior to uptake by the neuron.

For investigative work involving animals, wheat germ agglutinin (WGA) can be used to provide specificity for active uptake and transport. However, there are a wide range of nerve adhesion molecules which can be used to cause selective and active adsorptive endocytosis into nerves. This class of nerve adhesion molecules includes:
1) Anti-synaptosomal monoclonal (and non-monoclonal) antibodies which are purified or generated based upon their affinity for nerve membranes. These can be made by using crude nerve homogenates and then testing for endocytotic efficacy in cultured neuroblasoma cells or by direct measurement of uptake of radiolabeled forms after intramuscular injection in laboratory animals. These agents may involve entire antibodies of, preferably, the fragment of the antibody responsible for recognition without the F_{c} region. Similar considerations apply for antibodies to dopamine-beta-hydroxylase.
2) Various growth factors such as nerve growth factors, epidermal growth factors, insulin-related growth factors and other proteins and peptides in this functional category which are known to have or discovered to have efficacy at causing the neuronal uptake of themselves and of other agents with which they are conjugated.
3) Lectins of various sorts which are proteins having a high degree of affinity for particular carbohydrate and other types of cell surface markers. This is meant to include both plant lectins as well as various endogenous vertebrate, mammal, or human lectins as are known or may be discovered.
4) Fragments of neurotrophic viruses such as Herpes simplex, pseudorabies virus or poliovirus or proteins or other markers from the viral coat responsible for their highly efficacious uptake and transport. These fragments or inactivated whole viral particles, or cloned and produced copies of the crucial proteins are of particular interest for trans-neuronal transport.
5) Fragments of bacterial toxins such as the B-chain of cholera toxin and non-toxic fragments of tetanus toxin such as the C fragment as well as modified versions or cloned portions of other safely administered proteins with high neural affinity.
6) A wide variety of peptides and small proteins such as endorphins, vasoactive intestinal polypeptide, calcitonin gene-related peptide, cholecystokinin, substance P, somatostatin, and neuropeptide Y or the relevant portions of such peptides for the encouragement of neuronal uptake and transport.
7) Enzymes which are selectively endocytosed for synaptic recycling purposes such as acetylcholinesterase and dopamine beta hydroxylase or portions of these enzymes which are effective at inducing neuronal uptake.
8) Various cell adhesion molecules including peptides, proteins and various simple and complex carbohydrates which are effective at promoting neuronal uptake and transport of conjugated pharmaceutical agents.
9) Neurotransmitters and neurotransmitter analogs such as GABA, D-aspartate, dopamine, norepinephrine, serotonin, and benzodiazepine drugs which can be so constructed as to maintain their efficacy in promoting transport after the conjugation to pharmaceutically useful agents.

Two optimal nerve adhesion molecules for most applications are transferrin and β-nerve growth factor depending on whether primarily mixed or primarily sensory nerves are to be imaged; however, various other molecules may be optimal for particular sorts of pharmaceutical tasks.

These proteins and other nerve adhesion molecules can be attached to the therapeutic or prophylactic moiety or to the coating (e.g. dextran coating) thereof by various methods including a periodate oxidation reaction carried out under mild conditions which does not affect the oxidation state of ferrite crystals or the binding affinity of the targeting molecule. Other binding reactions include carbodiimide binding, glutaraldehyde binding, biotin/avidin linking, or noncovalent coating with the molecule of interest. Metal oxide particles provide a core of useful size which can be securely coated by a variety of types of agents under mild, non-denaturing conditions - they are therefore useful as a particle seed even when their special metallic or magnetic properties are not relevant to the task at hand.

All of the above conditions may be optimized so as to use this new drug administration method to efficiently deliver a specially designed pharmaceutical to a specific and preselected location within the nervous system. The sites will include peripheral nerves along their continuous lengths, sensory ganglia, autonomic ganglia, spinal cord, and brainstem or the olefactory tract system upon intranasal administration. When the route of administration is intrathecal, or topically upon the brain during surgery or in the brain during open surgery or during stereotactic surgical procedures, then a wide range of sites within the central nervous system can be reached. In these cases of direct application in the brain, it may be the purpose of the agent to be transported to a surgically inaccessible site by axonal transport from a surgically accessible site, or in any case to a site different from the site of application. This may also be used to demonstrate a successful incorporation of grafted neural tissue which will become capable of transporting included agents from the graft to distant sites. It will occasionally be helpful to achieve transport with a small molecule such as an amino acid or protein labelled with e.g. a positron emitting nuclide; however, in many of the applications, the preferred type of agent will include a particle which is up to 50 nanometers in diameter and may contain many atoms, crystal subunits, or molecules of the active ingredient for diagnosis or therapy.

The exact relation between particle core size and resulting T₂ relaxivity per microgram or iron is dependent on a variety of factors in the particle synthesis and upon the biochemical environment in which the particle must exist prior to imaging. Shen in SMRM 10:871 (1991) reports a relation between relaxivity and crystal size which greatly favours the use of the largest possible particle. Relaxivity data on particles produced by the inventor showed values of up to 4.0 x 10⁵ sec⁻¹M⁻¹ which are greater than those reported by Shen and are consistent with this trend. Thus, an optimal relaxation effect per amount of iron to be cleared by the nerve may be achieved with the largest particles endocytosed and transported efficiently.

When more diffuse effects are desired, an injection into the bloodstream or into the cerebro-spinal fluid can be used to cause uptake by a specific type of neuron wherever it contacts the fluid. However, for specific delivery to an individual peripheral nerve, the intramuscular technique will be preferred.

The usefulness of the intramuscular administration derives from the fact that individual axons which enter a muscle divide multiply and fan out to innervate numerous individual muscle cells making up a 'motor unit'. Such a motor unit is a group of muscle cells which fire simultaneously whenever their shared axon delivers a depolarization signal. Due to the dictates of muscle energetics and control mechanisms, the cells of a unit are generally scattered through a muscle rather than concentrated in a single area. Thus an injection at any one site will usually be near the axon termini of a large number of different motor units. This presents the injectate with a relatively large axon terminal surface area and provides access to a mix of axons distributed evenly through the incoming nerve. Additionally, because all muscles have a rich sensory supply to muscle spindle tension, length, and rate receptors, distributed through the muscle, there is simultaneous access to sensory and to motor fibers.

When I¹²⁵-NGF was injected in muscle, high intraneural concentrations were achieved because of uptake by the various types of sensory endings in the spindle organs of the muscle.

Intramuscular injections with intraneuronal pharmaceuticals present no greater risk of nerve injury than any other sort of routine intramuscular injection. However, it is well documented that any fine axonal branch which is injured causes no lasting muscle impairment. This is because of the short distance of regrowth and the tendency for axon terminal branches to recolonize any denervated muscle cell. It should be noted that although injury to some axonal branches may occur, the distribution of tracer in nerve and in cell bodies upon histologic inspection of the spinal cord is consistent with uptake by intact cells rather than by any important contribution from uptake by injured cells.

It is often necessary to administer drugs whose intended site of action is in the spinal cord or dorsal root ganglia (DRG). However, in the past there has been no easy way of safely administering such drugs near their site of action. While injections are commonly used to achieve local drug effects in muscle or in joints, it is exceedingly hazardous to introduce a needle percutaneously and blindly into the vicinity of the spinal cord or sensory ganglia. Therefore, in order to achieve pharmacologically efficacious doses of various drugs at these locations it has been necessary to give very high systemic doses by intravenous and oral routes, or by intramuscular routes wherein the actual delivery of the drug depended upon vascular uptake from the injection site in order to achieve the best available distribution. Alternatively, cumbersome procedures in which special catheters are threaded into place near the spinal cord have been undertaken with attendant dangers of spinal cord injury. There has never been any route except by whole body vascular distribution to deliver any drugs to ganglia.

However, by taking advantage of the drug distributions achievable by axonal transport where particulates are used, a dramatic change in pharmaceutical practice can be achieved. When the desired drug is trapped in a polymer, liposome, or protein nanosphere with an attached nerve adhesion molecule, it becomes possible to carry out an intramuscular injection of an extremely small amount at a location to which the desired part of the nervous system is connected by a peripheral nerve. Most of the injectate will stay in the muscle at the site of injection while the drug particles are ingested by nerve endings and transported to the ganglionic or spinal cord sites toward which treatment is to be directed.

Liposomes with phosphatidlycholine and phosphatidylethanolamine (e.g. as described by Grant in Mag. Res. Med. 11:236-243 (1989)), derivatized for attachment of a nerve adhesion molecule can be prepared in the necessary size range for efficient uptake and neuronal transport. In this fashion, a wide variety of hydrophilic and hydrophobic drugs can be packaged for delivery direct to their intended neural sites of action while minimizing systemic dose.

The particles yield a large amplification of the uptake process since many drug molecules are ingested by the cell with each event and helps minimize spread of the drug away from the injection site.

While there are a number of well known methods for producing particulate drug carriers, many of these are not suitable for intraneural drug delivery. Many of the known techniques are not applicable because they result in the production of particles which are far too large to permit access to the axon terminus in general and synaptic cleft in particular as needed to promote neural uptake. For instance, many polylactic acid particles can only be produced in sizes near 100 microns which is three orders of magnitude too large. Liposomes similarly tend to be too large unless they are produced as small unilamellar vesicles (SUV) which require harsh physical and chemical conditions for synthesis.

Polycyanoacrylate/vinyl particulates (many of which are termed "latex" microspheres or nanoparticles) can be readily produced in the appropriate size range, but require the use of organic solvents and other treatments which are destructive to many biological molecules of therapeutic interest. Albumin or other protein microspheres of useful size can be produced from sonicated emulsions, but these require denaturing by heating over 100°C or chemical crosslinking to achieve stability and this similarly limits the range of potential pharmaceuticals.

Metal oxide particles are particularly convenient as drug carriers. They readily form stable colloids of appropriate size and can be coated by a wide variety of molecules. When the particles are prepared by adding a strong base to the metal chlorides in saturated dextran, a strong bond between the particle and the dextran is formed. Subsequently, some proteins can be covalently bound to the dextran molecules. However, this type of synthesis precludes the use of any drugs which cannot tolerate the strong acidity of the metal salts or the rapid shift to a strong alkali typically used in the precipitation reaction.

The predominant solution to this problem has been to precipitate the particles with no coating, and then apply the coating at a later step under more mild conditions. However, whether NaOH or NH₄OH is used, and whether the metal salts are added to the alkali or vice versa, uncoated particles always aggregate and despite high power sonication of the resuspended pellets, it is difficult to produce a stable colloid.

Precipitation by addition to ammonia is more effective than using NaOH because of a clear peptizing effect reconfirmed in experiments conducted by the inventor. Such uncoated ammonia precipitates become stable for centrifugal ultrafiltration even without sonification. This preparation is sufficiently reactive as to permit reliably stable but non-covalent binding of a wide range of molecules. In one series of preparations made by the inventor, coatings were made with alpha-cyclodextrin which is capable of binding relatively non-polar drugs and is well known as a drug vehicle. Further, particles were prepared which were first used to adsorb tritiated dexamethasone and then to adsorb WGA. These particles were finally coated with dextran or bovine serum albumin to cover unused reactive sites on the particle surface. Preparations of this sort were subjected to repeated washing, ultrafiltration, and N-acetylglucosamine affinity purification. These steps demonstrated a very slow release of the labelled dexamethasone, but also showed that the affinity purified particles carried with them a high concentration of bound dexamethasone.

Particles of this sort can be used to introduce this helpful steroid drug into neurons for delivery to areas of spinal cord injury. As has also been shown by the inventor, such particles are extruded from the neuron into the endoneurial space and so are then placed in a position to properly interact with and activate cell surface glucocorticoid receptors at otherwise inaccessible locations inside the blood brain barrier.

An entirely novel means of producing metal oxide particles under mild conditions suitable for delicate proteins and peptides has also been developed by the inventor. This method is based on the tendency of mixed iron salts to commence precipitation when the pH is raised above 4.0. Thus, rather than raising the pH of the solution to pH 9, 10 or 11 as is done in all previously known methods, the new method involves precipitating the crystals at physiological pH in biochemically tolerable buffer solutions.

Thus a suitable method of producing a physiologically tolerable particulate metal oxide comprises precipitating said oxide from a biologically tolerable, physiological pH buffered solution, optionally containing a coating agent whereby coated particles are formed.

For instance, a strong buffer such as 1 molar HEPES pH 7.4 is prepared with the desired coating molecules in the buffer solution. The mixed metal salt solution, either with or without dextran, is then added to the buffer solution in dropwise fashion. This method has resulted in the production of stable coated colloids produced at physiological pH. This new method of production of these crystals greatly broadens the range of pharmaceuticals which can be included in the particle coat of these 10-100 nm particles for delivery by intraneural or other routes.
Treatment of HTLV-I Associated Myelopathy: A focal infection of the spinal cord associated with HTLV-1 (Human T-Cell Leukaemia Virus, type 1) in which there is involvement of the phagocytic microglia and oligodendrocytes as well as of neurons has presented a very forbidding picture for any possible treatment options. What few anti-viral agents have been available have poor penetration of the blood brain barrier and so must be administered intravenously in very high concentration to achieve potentially therapeutic dose levels in the spinal cord. Similarly, intrathecal administration into the CSF allows uncontrolled spread of the anti-viral agent throughout the CNS, affecting various sites of reduced blood brain barrier (e.g. median eminence of the hypothalamus, pineal gland, and area prostrema) more than the infected site. Further, intrathecally administered drugs tend to be swept out of the CSF into the blood stream over a relatively rapid time course compared to what is required for anti-viral therapy.

However, because of the focal nature of the lesion, it is quite easy to identify the dermatome supplied by the involved location of the spinal cord. Therefore, by undertaking intramuscular injections in neck/back muscles as well as in muscles placed at greater distances from the spinal cord, it is possible to set up a continuous inflow of specific medication into the CNS neurons, and by transcellular transport, into the surrounding oligodendrocytes and microglia. The active agents used can include small molecules conjugated to a nerve adhesion molecule, small liposomes or other types of nanoparticles carrying antiviral drugs. The administration of steroids by the same route can also help to reduce injury due to inflammatory components of the disease.

Where transport in the affected dermatome is severely impaired by extensive cell death, injections on the contralateral side of the body and at dermatomes below and above those affected will still bring the drugs into a relatively high concentration at close proximity to the lesion and usefully across the blood brain barrier.
Pain: Among the most common of all tasks faced by the physician or surgeon is the treatment of a severe localized pain whose duration will probably be only a few days but which will cause considerable distress and discomfort to the patient. This might be from a bone fracture, ankle sprain, a surgical incision, abscess, severe back muscle spasm, exacerbation of arthritis, dental extraction, burn, or tumour among other problems. The available pharmaceutical options at present fall into four main categories, an oral drug such as aspirin or acetaminophen which acts at the site of injury by altering the effects of prostacyclin related pain mediators, a range of oral or locally injected anti-inflammatory drugs of both steroidal and non-steroidal composition, locally injected anaesthetics such as marcaine or lignocaine which reduce neural activity, and the systemic administration of opiates or opiate analogs by oral, intramuscular or intravenous routes. There are a few extraordinary treatments most commonly undertaken in cancer patients such as the placement of continuous infusion morphine cannulas near the spinal cord but these are little used because of difficulty in preventing infection and the complexity of placing them. Further, such treatments cannot be used for pain in the arm or neck because free opiates in spinal fluid of the upper spinal cord may reach the medulla and cause respiratory arrest.

If opiate or anti-inflammatory drugs are included in particles, bound to nerve adhesion molecules, and then injected in the dermatome/myotome which is involved in the pain, then an extremely efficient distribution can be achieved. This permits the extended administration of an opiate at an extremely high concentration in the dorsal root ganglion and dorsal root entry zone of the spinal cord at the involved level, while resulting in negligible systemic levels of the drug. For opiates, this avoids tolerance, sedation, respiratory depression and addiction and provides for steady long term administration over days after a single injection. For steroids and non-steroidal anti-inflammatory drugs, this will help reduce the risk of gastric irritation and internal bleeding as well as the other side effects of steroids.

Some types of chronic pain syndromes are remarkably resistant to all standard pain medications including the various opiates and opiate derivatives. The prototype for this sort of pain is trigeminal neuralgia and indeed the sine qua non of this syndrome is that the pain can be relieved only by anticonvulsant medications such as carbamazepine. This sort of chronic pain is often viewed as a kind of focal sensory epilepsy. The problem in treating such pain is that there is often great difficulty in achieving adequate doses at the dorsal root entry zone of the spinal cord or the trigeminal nuclei in the brainstem without causing unacceptable systemic side effects. By use of pharmaceutical agents according to the invention, agents such as carbamazepine can be delivered via an intraneural route after intramuscular injection or intradermal injection.
Spinal Cord Injury: Although acute spinal cord injury has long proven very difficult to treat, it has recently been appreciated that extremely high doses of methylprednisolone given intravenously over the first 24 to 48 hours post-injury can be significantly beneficial to eventual neurological outcome. Of course, the actual site of action is at the injury location in the spinal cord, but the dose is limited by the extremely large amounts of steroid which must be distributed to the rest of the body for no useful purpose.

These steroids can be incorporated into liposomes or other particles of appropriate size, conjugated to a nerve adhesion molecule and injected intramuscularly in back or neck muscles at the level of sensory or motor loss. When this is done, they are transported directly into the nervous system at a continuing flow and arrive precisely at the site of injury. Wherever actual nerve compression or injury has occurred, the transported agent will accumulate and automatically achieve particularly high concentrations. Drug leaking out of torn or inflamed cells, and extruded into the nervous tissue around the affected neurons will act on other injured tissues in the vicinity. This will also assure good delivery into the spinal cord grey matter even when the fracture has caused venous congestion which is slowing vascular delivery to the most affected areas.

Certain pharmaceutical agents useful in the method according to the invention are already known - others may be produced by methods analogous to those used for producing the known agents, e.g. for particulate agents: obtaining particles of a matrix material comprising a physiologically active agent or diagnostic marker; optionally coating said particles with a physiologically tolerable optionally biodegradable coating material, e.g. a natural or synthetic polymer or derivative thereof such as latex, polylactic acid, proteins, albumin, polysaccharides, starches, dextrans, polymerized sugar alcohols, etc (see for example EP-A-184899 (Jacobsen)); and conjugating said particle (optionally via coupling to a said coating, optionally after appropriate derivatization thereof e.g. to provide a binding site or to block excess binding sites) to a nerve adhesion molecule, preferably with a NAM: particle ratio of up to 10, especially up to 5 more especially up to 2 and most preferably about 1; optionally separating NAM-conjugated particles so formed from unconjugated particles, preferably by size separation, especially preferably by repeated size separation followed by at least one affinity separation; optionally sterilizing the NAM-conjugated particles, if desired after formulation thereof with a pharmaceutical carrier and optionally with further conventional pharmaceutical excipients, e.g. viscosity enhancing agents, pH regulators, osmolality adjusting agents, etc.

The matrix material used may be an inorganic matrix, e.g. a metal oxide, or an organic matrix, e.g. a polymer such as a cross-linked starch or dextran, and it may serve as a carrier for the physiologically active agent or diagnostic marker or it may itself serve as the active agent or marker, as would for example be the case with superparamagnetic ferrite crystals.

Incorporation of the agent or marker within a carrier matrix can be achieved by conventional techniques, for example by co-precipitation, by steeping a porous matrix material to impregnate it with the desired agent or marker, by exposing the agent to ultrasonically suspended, uncoated metal oxide particles, or by means of the buffered precipitation technique described herein.

The matrix particles should desirably be relatively uniformly dimensioned, e.g. within the ranges discussed above, and this may be achieved for example by conventional screening or particle precipitation techniques. Monodisperse particles will be preferred.

Where the agent used according to the invention is non-particulate it may again be produced by conventional techniques, e.g. by binding a desired nuclide directly or via a chelant molecule to a NAM or by binding a chromophore or fluorophore or a physiologically active molecule to a NAM, optionally and indeed preferably so as to provide a biodegradable bonding which will permit liberation of the physiologically active agent after endocytosis.

For axonal delivery of therapeutic or prophylactic substances, in certain cases it may be desirable in the method of the invention to select physiologically active substances which occur naturally in neurons or which are analogues of such naturally-occurring substances.

A suitable process for the preparation of the modified spinel and garnet particles used in the invention comprises precipitating di and trivalent metal ions of ionic radii such as to permit crystals of spinel or garnet structure to form, said precipitation being from a solution containing scandium, a radioactive yttrium isotope, a sixth period metal, a high MR receptivity nuclide or an element having a desired therapeutic or prophylactic activity.

In these particle precipitation processes the physiologically active, marker, or high positron affinity elements to be incorporated into the particles may themselves be in solution or alternatively they may be in fine "seed" crystals which become included in the precipitating particles.

For administration in vivo, the dosages used will clearly depend upon a wide range of factors such as the patient's weight, the specificity of the NAM (for NAM-conjugated agents), the nature of the physiologically active or diagnostic marker component of the pharmaceutical agent, the extent or severity of the injury or ailment that is being investigated or treated, the distance over which axonal transport is required, etc. The appropriate dosage however can readily be determined taking these factors into account.

However the intramuscular administration according to the invention of NAM-targetted agents offers the possibility of very efficient and very specific delivery.

## Claims

1. Use of a therapeutic agent comprising particles having, coupled thereto, a nerve adhesion moiety and a physiologically-active substance, the nerve adhesion moiety serving to promote neuronal endocytosis of said agent, the agent being capable of axonal transport following neuronal endocytosis, for the preparation of a medicament for providing the therapeutic effect of the physiologically-acceptable substance on administration into a vascularised peripherally innervated tissue site or into other tissue sites innervated by a spinal root.

2. Use as claimed in claim 1, wherein the agent has a mean particle size of 5-100 nm.

3. Use as claimed in claim 2, wherein the particle size is 10-50 nm.

4. Use as claimed in any preceding claim, wherein the particles have a spinel structure.

5. Use as claimed in any preceding claim, wherein the particles are superparamagnetic.

6. Use as claimed in any preceding claim, wherein the particles incorporate radionuclides.

7. Use as claimed in any preceding claim, wherein the particles are coated with dextran.

## Patentansprüche

1. Verwendung eines therapeutischen Mittels, welches Teilchen umfaßt, an die eine Nervadhäsions-Einheit und eine physiologisch aktive Substanz gekoppelt sind, wobei die Nervadhäsions-Einheit dazu dient, eine neuronale Endozytose des Mittels zu fördern, wobei das Mittel im Anschluß an die neuronale Endozytose zu einem Neurit-Transport in der Lage ist, zur Herstellung eines Medikaments für die Bereitstellung der therapeutischen Wirkung der physiologisch akzeptablen Substanz bei Verabreichung in eine vaskularisierte, peripher innervierte Gewebestelle oder in andere durch eine Spinalwurzel innervierte Gewebestellen.

2. Verwendung nach Anspruch 1, worin das Mittel eine mittlere Teilchengröße von 5 - 100 nm aufweist.

3. Verwendung nach Anspruch 2, worin die Teilchengröße 10 - 50 nm beträgt.

4. Verwendung nach irgendeinem vorhergehenden Anspruch, worin die Teilchen eine Spinellstruktur aufweisen.

5. Verwendung nach irgendeinem vorhergehenden Anspruch, worin die Teilchen superparamagnetisch sind.

6. Verwendung nach irgendeinem vorhergehenden Anspruch, worin die Teilchen Radionuklide enthalten.

7. Verwendung nach irgendeinem vorhergehenden Anspruch, worin die Teilchen mit Dextran beschichtet sind.

## Revendications

1. Utilisation d'un agent thérapeutique comprenant des particules auxquelles sont couplés un groupement d'adhésion neurale et une substance physiologiquement active, le groupement d'adhésion neurale servant à activer l'endocytose neuronale de cet agent, agent qui est apte au transport axonal après endocytose neuronale, pour la préparation d'un médicament destiné à engendrer l'effet thérapeutique de la substance physiologiquement acceptable par administration dans un site tissulaire vascularisé à innervation périphérique ou dans d'autres sites tissulaires innervés par une racine spinale.

2. Utilisation suivant la revendication 1, dans laquelle l'agent a un diamètre moyen de particules de 5 à 100 nm.

3. Utilisation suivant la revendication 2, dans laquelle le diamètre de particules est compris dans l'intervalle de 10 à 50 nm.

4. Utilisation suivant l'une quelconque des revendications précédentes, dans laquelle les particules ont une structure de spinelle.

5. Utilisation suivant l'une quelconque des revendications précédentes, dans laquelle les particules sont des particules supraparamagnétiques.

6. Utilisation suivant l'une quelconque des revendications précédentes, dans laquelle les particules renferment des radionucléides.

7. Utilisation suivant l'une quelconque des revendications précédentes, dans laquelle les particules sont enrobées avec du dextrane.
